# EUROPÄISCHE PATENTSCHRIFT

(11) **EP 2 164 567 B1**
(45) Veröffentlichungstag und Bekanntmachung des Hinweises auf die Patenterteilung: **06.01.2016**
(21) Anmeldenummer: 08733825.7
(22) Anmeldetag: 30.04.2008
(51) Int. Cl.: A61N 5/06

(54) **TRAGBARE BESTRAHLUNGSANORDNUNG**
PORTABLE IRRADIATING ARRANGEMENT
DISPOSITIF DE RADIATION PORTABLE

(30) Priorität: 29.06.2007 CH 10512007
(43) Veröffentlichungstag der Anmeldung: 24.03.2010
(73) Patentinhaber: Alplight, 6460 Altdorf (CH)
(72) Erfinder: BISSIG, Alois, 6454 Flüelen (CH); ZURFLUH, Erich, 6460 Altdorf (CH)
(74) Vertreter: Fischer, Britta Ruth
(86) Internationale Anmeldenummer: PCT/CH2008/000199
(87) Internationale Veröffentlichungsnummer: WO 2009/003295

(56) Entgegenhaltungen:
- EP-A- 1 433 499
- WO-A1-93/16323
- JP-A- 7 323 093
- US-A1- 2003 210 542
- US-B1- 7 029 193

## Beschreibung

Die Erfindung betrifft eine tragbare Bestrahlungsanordnung mit einer Lichtquelle gemäss Oberbegriff des unabhängigen Anspruchs 1. Als Lichtquelle wird ein Laser bzw. eine Laserdiode oder auch ein Lichtwellenleiter eingesetzt. Die tragbare Laseranordnung kann beispielsweise auf dem Gebiet der Medizintechnik, insbesondere für Belichtungszwecke, eingesetzt werden, ist jedoch nicht auf Anwendungen auf diesem Gebiet beschränkt.

### Stand der Technik

Bestrahlungsanordnungen werden in der Medizintechnik in vielerlei Hinsicht angewendet. Bestrahlungsanordnungen mit einem Laser als Lichtquelle, so genannte Laseranordnungen, werden beispielsweise in der photodynamischen Therapie (PDT), der photodynamischen Desinfektion (PDD) und in der Low-Level Light Therapy (LLLT) eingesetzt. Ferner werden Laseranordnungen auch zum Schneiden von Gewebe, Bohren in bzw. an Zähnen, zur endodontischen Behandlung, in der Veterinärmedizin, zum Aushärten von Zahnfüllungen, zum Zahnweissen und ähnlichem eingesetzt. Für bestimmte der aufgezählten Anwendungen ist es vorteilhaft, eine tragbare Laseranordnung einzusetzen.

Bekannte tragbare Laseranordnungen mit Lichtleistungen im Bereich von mehr als 1 Milliwatt bis über 200 Milliwatt sind häufig so ausgelegt, dass die Strahlung ungehindert austreten kann und somit eine Gefahr für den Benutzer und den Patienten darstellen kann. Derartige Laser sind gemäss den Laser-Klassifizierungen IEC 60825-1/IEC 601-2-22 bzw. der entsprechenden ANSI-Norm(en) der Laserklasse 3B bzw. 4 zugeordnet. Der Laserklasse 3B sind Laseranordnungen zugeordnet, deren zugängliche Laserstrahlung gefährlich für das Auge und in besonderen Fällen auch für die Haut ist, wobei diffuses Streulicht in der Regel ungefährlich ist. Der Laserklasse 4 sind Laseranordnungen zugeordnet, deren zugängliche Laserstrahlung sehr gefährlich für das Auge und gefährlich für die Haut ist, wobei auch diffuses Streulicht gefährlich sein und die Laserstrahlung Brand- oder Explosionsgefahr verursachen kann (vergleiche http://de.wikipedia.org/wiki/Laser). Für Laseranordnungen dieser Laserklassen sind gemäss der obengenannten Laser-Klassifizierungen Schutzvorrichtungen vorzusehen, wie beispielsweise ein Sicherheitsschloss, ein Sicherheits"Interlock"-Stecker, eine "Laser an"-Anzeige, eine "Laser bereit"-Anzeige, eine redundante Timer-Elektronik, ein "Notaus"-Schalter usw. Des Weiteren müssen Benutzer und Patient Laserschutzbrillen tragen, die mitunter das Sichtfeld beeinträchtigen können, wobei die Kosten der Laserschutzbrillen mit der Gefährlichkeit der Laseranordnung und somit mit der Laserklasse zunehmen. Bei bekannten Laseranordnungen der obengenannten Laserklassen sind also zusätzliche, oftmals kostenintensive Schutzvorrichtungen erforderlich.

Bei vielen, insbesondere medizintechnischen Anwendungen von Laseranordnungen kommt es oftmals zu Verunreinigungen der Laseranordnung durch Blut, Speichel, Bakterien, Gewebe, usw. Bekannte Laseranordnungen aus dem Stand der Technik sind oftmals schwierig zu reinigen und steril zu halten, beispielsweise indem sie in einer Autoklave mit heissem Dampf bei 135°C sterilisiert werden. Aus US 7 029 193 B1 ist eine Laserzeigevorrichtung bekannt, die auch für Beleuchtungszwecke verwendet werden kann. Aus JP 07 323093 A und aus EP 1 433 499 A sind medizinische Bestrahlungsvorrichtungen mit Laser bekannt. US 2003/0210542 A1 und WO 93/16323 zeigen Taschenlampen.

### Darstellung der Erfindung

Der vorliegenden Erfindung liegt die Aufgabe zugrunde, eine tragbare Bestrahlungsanordnung mit einer Lichtquelle zu schaffen, die bei einer als Laser bzw. Laserdiode ausgeführten Lichtquelle einer kleineren bzw. ungefährlicheren Laserklasse als 3B zuzuordnen ist. Insbesondere soll die erfindungsgemässe tragbare Bestrahlungsanordnung bei einer als Laser bzw. Laserdiode ausgeführten Lichtquelle einer Laserklasse von 2M bzw. einer darunter liegenden, ungefährlicheren Laserklasse zuzuordnen sein. Bei der Laserklasse 2M liegt die zugängliche Laserstrahlung nur im sichtbaren Bereich (400 Nanometer bis 700 Nanometer). Sie ist bei kurzzeitiger Strahlungsdauer (bis 0.25 Sekunden) auch für das Auge ungefährlich, solange keine optischen Instrumente, wie Lupen oder Ferngläser, verwendet werden (vergleiche http://de.wikipedia.org/ wiki/Laser).

Es ist ferner Aufgabe der vorliegenden Erfindung, eine tragbare Bestrahlungsanordnung bereitzustellen, die einfach und effektiv gereinigt und sterilisiert werden kann.

Die Aufgabe wird durch eine tragbare Bestrahlungsanordnung mit den Merkmalen des Anspruchs 1 gelöst.

Die erfindungsgemässe tragbare Bestrahlungsanordnung umfasst eine Lichtquelle, bei der es sich um einen Laser bzw. eine Laserdiode handelt, eine Aussenhülse, eine Innenhülse und eine Strahlungsverteilersonde, wobei die Innenhülse in der Aussenhülse gelagert ist und die Strahlungsverteilersonde an einem Ende der Aussenhülse anbringbar ist. Das Ende der Aussenhülse, an dem die Strahlungsverteilersonde anzubringen ist, wird auch als sondenseitiges Ende der Aussenhülse bezeichnet. Die erfindungsgemässe Bestrahlungsanordnung ist derart ausgestaltet, dass die Lichtquelle nur dann einschaltbar ist, wenn die Strahlungsverteilersonde an der Aussenhülse angebracht ist. Das heisst, die erfindungsgemässe Bestrahlungsanordnung kann nur dann von einem Benutzer eingesetzt werden, wenn die Strahlungsverteilersonde an der Aussenhülse bzw. an dessen sondenseitigem Ende angebracht ist. Als Lichtquelle kann auch ein Lichtwellenleiter, insbesondere eine Lichtwellenleiter-Faser, eingesetzt werden.

Gemäss bevorzugter Ausgestaltung der Erfindung ist eine Federung vorgesehen, die in axialer Richtung auf die Innenhülse wirkt, sodass ein erster Abschnitt der Innenhülse zumindest teilweise aus dem sondenseitigen Ende der Aussenhülse herausragt, wobei die Strahlungsverteilersonde derart ausgebildet ist, dass sie im angebrachten Zustand den aus der Aussenhülse herausragenden Teil des ersten Abschnitts der Innenhülse gegen die Federkraft der Federung zumindest teilweise in die Aussenhülse drückt bzw. zurückdrückt.

An der Aussenhülse ist vorzugsweise ein Bedienelement, insbesondere ein Druckknopf oder ein Berührfeld, zum Einschalten der Lichtquelle vorgesehen und die Innenhülse weist vorzugsweise eine Öffnung auf, wobei der axiale Abstand zwischen dem Bedienelement und der Öffnung derart gewählt ist, dass nur bei angebrachter Strahlungsverteilersonde das Bedienelement durch die Öffnung in Wirkverbindung mit der Lichtquelle treten kann.

Zusätzlich oder alternativ ist in der Aussenhülse vorzugsweise ein Sensor vorgesehen, der ein Angebrachtsein der Strahlungsverteilersonde erkennt.

Auf diese Weise wird sichergestellt, dass die erfindungsgemässe tragbare Bestrahlungsanordnung nur bei angebrachter Strahlungsverteilersonde in Betrieb genommen bzw. dessen Lichtquelle eingeschaltet werden kann. Dies führt zu einer Einstufung der erfindungsgemässen tragbaren Bestrahlungsanordnung in eine Laserklasse, die für den Benutzer relativ sichere Laseranordnungen vorgesehen ist. Gegebenenfalls vorgesehene Schutzvorrichtungen können weniger aufwändig ausfallen als bei den aus dem Stand der Technik bekannten Laseranordnungen der Laserklassen 3B und 4 oder ganz wegfallen, sodass der Einsatz der erfindungsgemässen tragbaren Bestrahlungsanordnung kostengünstig ist.

In einer Ausführungsform steht die Innenhülse derart in Kontakt mit der Strahlungsverteilersonde, dass vor einem Entfernen der Strahlungsverteilersonde von der Aussenhülse die Innenhülse von der Strahlungsverteilersonde gelöst werden muss. Die Innenhülse verhindert insbesondere, dass die Strahlungsverteilersonde durch Drehen von der Aussenhülse entfernt wird.

Gemäss bevorzugter Ausgestaltung weisen die Innenhülse an einem ersten, sondenseitigen Abschnitt ein Aussenprofil und die Strahlungsverteilersonde ein dazu passendes Innenprofil auf, wobei das Innenprofil bei angebrachter Strahlungsverteilersonde auf dem Aussenprofil sitzt. Das Aussenprofil der Innenhülse ist vorzugsweise als Aussensechskant und das Innenprofil der Strahlungsverteilersonde ist vorzugsweise als dazu passender Innensechskant ausgeführt.

Dies hat den Vorteil, dass die erfindungsgemässe tragbare Bestrahlungsanordnung leicht zu reinigen und zu sterilisieren ist. Hierzu kann die Aussenhülse in einer so genannten Autoklave gereinigt und sterilisiert werden. Die Innenhülse, in der vorzugsweise die Lichtquelle, eine Stromversorgung für die Lichtquelle und elektronische Bauelemente für die Ansteuerung der Lichtquelle vorgesehen sind, ist während des Reinigungs- und Sterilisierungsprozesses durch die Aussenhülse und die Strahlungsverteilersonde geschützt, da die Strahlungsverteilersonde erst nach Entfernen der Innenhülse von der Aussenhülse entfernt werden kann. Inwieweit die Innenhülse sterilisierbar bzw. autoklavierbar ist, hängt insbesondere von der Ausgestaltung ihrer elektronischen Bauelemente ab. Ist es auch Sicherheitsgründen erforderlich, so kann die Innenhülse auch vor dem Sterilisieren bzw. Autoklavieren aus der Aussenhülse entfernt werden.

### Kurze Beschreibung der Zeichnungen

Weitere vorteilhafte Ausgestaltungen der Erfindung ergeben sich aus den Unteransprüchen und den anhand der Zeichnungen nachfolgend dargestellten Ausführungsbeispielen. Es zeigen
Figur 1 eine Darstellung einer erfindungsgemässen tragbaren Bestrahlungsanordnung,
Figur 2 eine Explosionsdarstellung der Darstellung in Figur 1,
Figur 3 eine Seitenansicht einer erfindungsgemässen tragbaren Bestrahlungsanordnung mit unterschiedlichen Strahlungsverteilersonden,
Figur 4 eine Darstellung einer erfindungsgemässen tragbaren Bestrahlungsanordnung mit entfernter Strahlungsverteilersonde,
Figur 5 eine Darstellung eines eine Löseeinrichtung aufweisenden Verschlusses der erfindungsgemässen tragbaren Bestrahlungsanordnung für das Lösen der Strahlungsverteilersonde,
Figuren 6 und 7 eine Darstellung eines Ausführungsbeispiels einer erfindungsgemässen tragbaren Bestrahlungsanordnung in Rückansicht (Figur 6a), als Querschnitt mit entfernter Strahlungsverteilersonde (Figur 6b), als Ausschnitt mit entfernter Strahlungsverteilersonde (Figur 6c) und als Ausschnitt mit angebrachter Strahlungsverteilersonde (Figur 7),
Figur 8 einen Ausschnitt einer erfindungsgemässen tragbaren Bestrahlungsanordnung mit einem Verschluss an ihrem hinteren Ende,
Figur 9 einen Querschnitt eines weiteren Ausführungsbeispiels einer erfindungsgemässen tragbaren Bestrahlungsanordnung mit entfernter Strahlungsverteilersonde (Figur 9a) und einen Ausschnitt hiervon (Figur 9b).

In den Figuren bezeichnen gleiche Bezugszeichen strukturell bzw. funktionell gleich wirkende Komponenten. Die Figuren erheben keinen Anspruch auf eine massstabsgerechte Darstellung.

### Weg(e) zur Ausführung der Erfindung

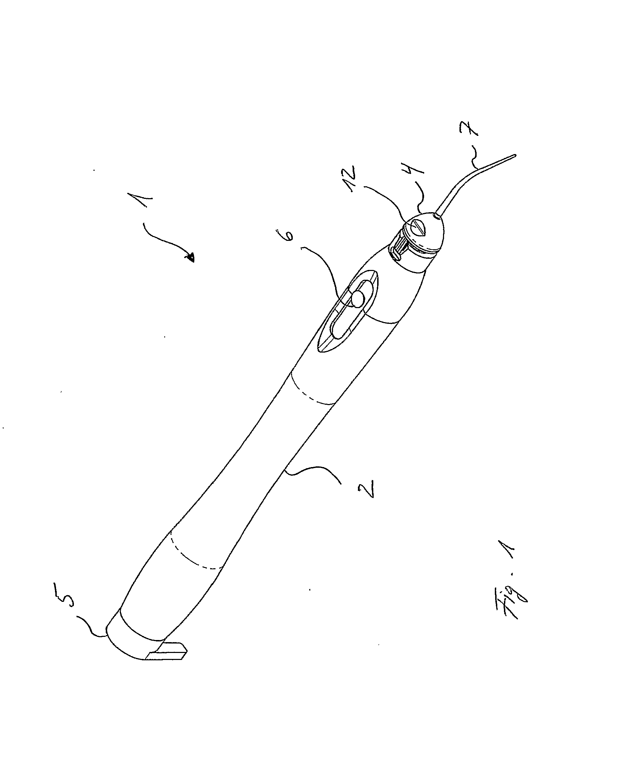

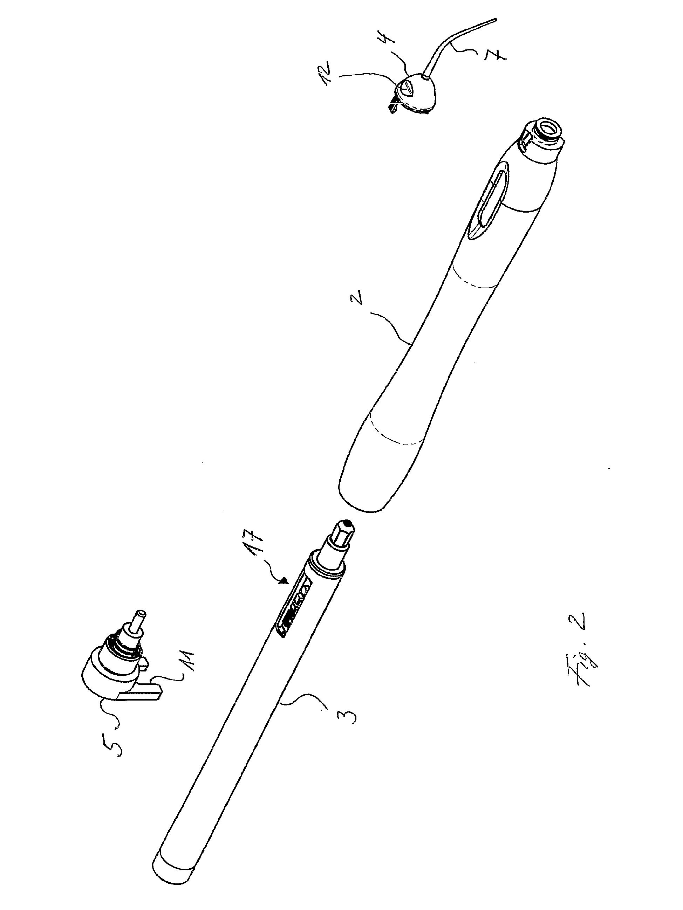

Die Figuren 1 und 2 zeigen eine erfindungsgemässe tragbare Bestrahlungsanordnung 1 in Zusammenbau- und Explosionsdarstellung. Die tragbare Bestrahlungsanordnung 1 umfasst eine Aussenhülse 2 und eine Innenhülse 3, die in der Aussenhülse 2 lösbar angeordnet ist. An dem einen Ende der Aussenhülse 2 ist eine Strahlungsverteilersonde 4 lösbar angebracht, insbesondere mittels einer Steckverbindung, wobei nicht dargestellte Widerhaken vorgesehen sein können, die beim Drehen abbrechen. Selbstverständlich ist es auch möglich, die Strahlungsverteilersonde 4 mittels einer Schraubverbindung an die Aussenhülse 2 anzubringen. Dieses Ende der Aussenhülse 2 wird entsprechend auch als sondenseitiges Ende der Aussenhülse bezeichnet. An dem diesem sondenseitigen Ende der Aussenhülse gegenüberliegenden Ende der Aussenhülse 2 ist ein Verschluss 5 lösbar angebracht.

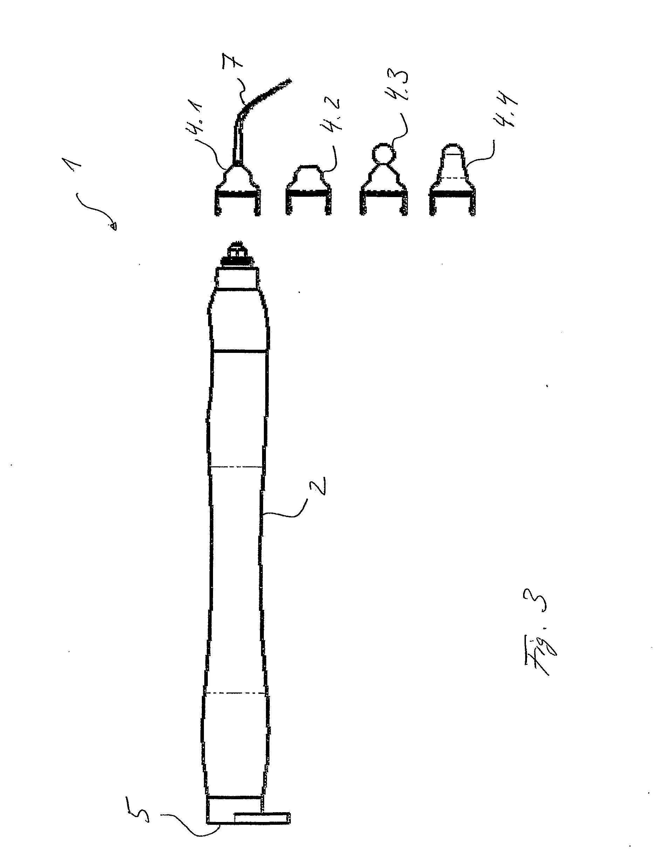

Die Strahlungsverteilersonde 4 streut oder absorbiert die Strahlung vorzugsweise in der Art, dass die austretende Strahlung eine Leistungsdichte hat, welche für Haut und Augenlicht eines Benutzers und eines Patienten ungefährlich ist. Die Strahlungsverteilersonde 4 kann einen Lichtleiter 7 aufweisen (vergleiche auch die Strahlungsverteilersonde 4.1 in Figur 3). Weitere mögliche Ausgestaltungen der Strahlungsverteilersonde 4 sind als Strahlungsverteilersonde 4.2, 4.3 und 4.4 in Figur 3 dargestellt.

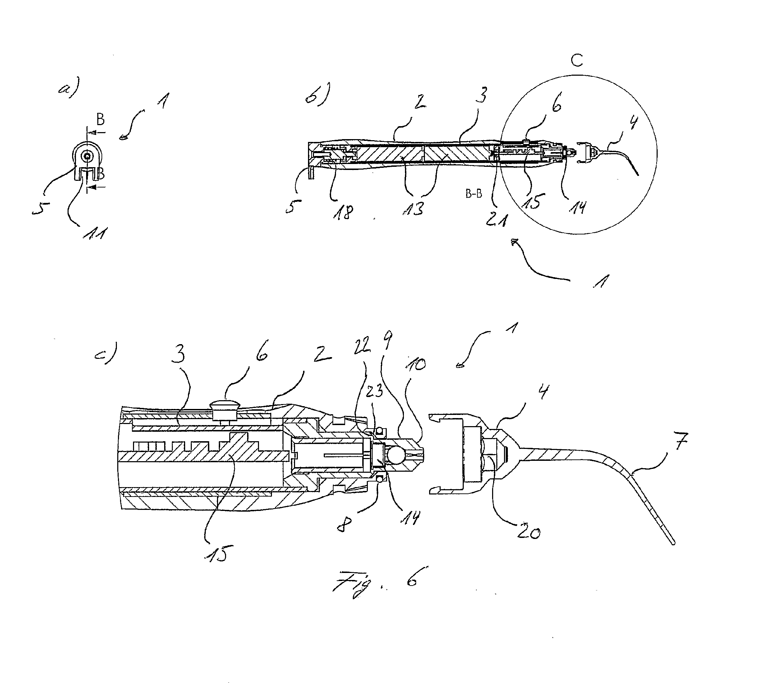

Es ist ein Bedienelement 6 zum Einschalten der in der Innenhülse 3 der tragbaren Bestrahlungsanordnung 1 angeordneten Lichtquelle 14 (vergleiche Figuren 6, 7 und 9) vorgesehen, das vorzugsweise als Druckknopf, Berührfeld, Schalter und/oder Taster ausgeführt ist. Zusätzlich oder alternativ kann das Bedienelement 6 kapazitiv, induktiv und/oder optisch wirkend ausgebildet sein. Das Bedienelement 6 ist vorzugsweise in bzw. an der Aussenhülse 2 angeordnet. Das Bedienelement 6 kann auch entfernt von der Bestrahlungsanordnung 1 als Fernbedienung ausgeführt sein, z.B. als Fussschalter, der bei Betätigung beispielsweise über Funk, Ultraschall und/oder mittels Infrarot ein entsprechendes Signal an die elektronischen Bauelemente 15 der Bestrahlungsanordnung 1 sendet.

Die Innenhülse 3 weist eine Öffnung 17 auf, wobei bei angebrachter Strahlungsverteilersonde 4 das Bedienelement 6 durch die Öffnung 17 in Wirkverbindung mit der in der Innenhülse 3 angeordneten Lichtquelle 14 (vergleiche Figuren 6, 7 und 9) treten kann. Durch Betätigung des Bedienelements 6 kann dann die Lichtquelle 14 bzw. die tragbare Bestrahlungsanordnung 1 eingeschaltet werden.

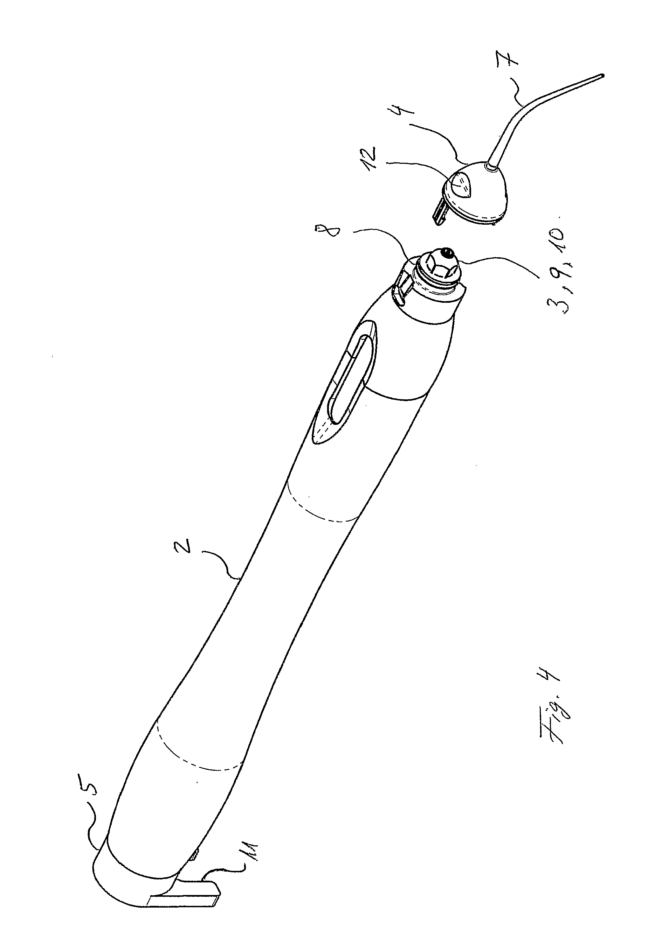

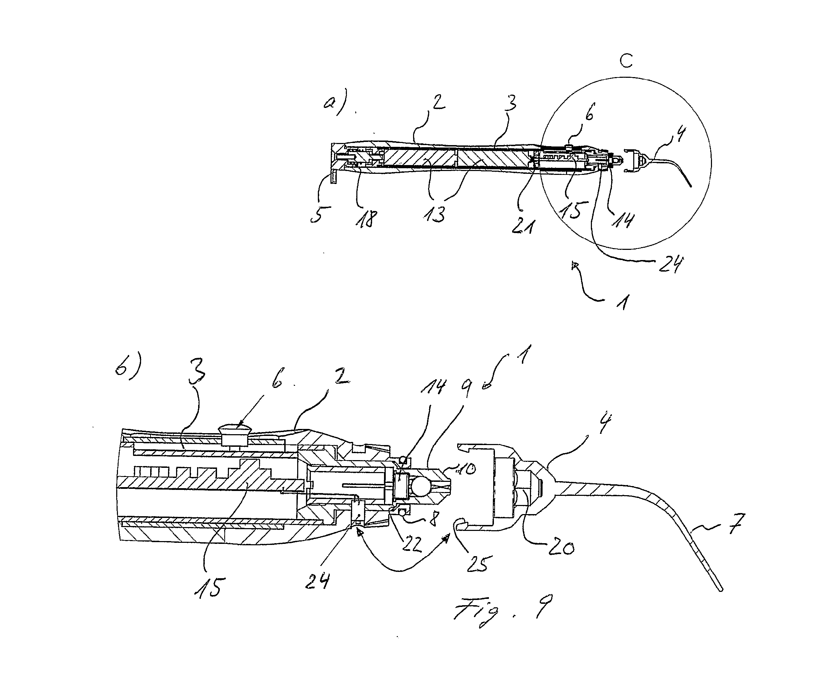

Die Innenhülse 3 ist, vorzugsweise mittels des Verschlusses 5, drehfest in der Aussenhülse 2 gelagert. Wie in Figur 4 dargestellt ragt die Innenhülse 3 mit einem ersten, sondenseitigen Abschnitt 9 an dem sondenseitigen Ende der Aussenhülse 2 aus dieser heraus. Zumindest an einem Teil dieses ersten, sondenseitigen Abschnitts 9 ist an dessen Umfang ein Aussenprofil 10, das vorzugsweise als Aussensechskant ausgebildet ist, vorgesehen. Die Strahlungsverteilersonde 4 weist ein zu diesem Aussenprofil 10 passendes Innenprofil 20 (vergleiche Figuren 6c und 9b) auf, sodass das Innenprofil 20 bei angebrachter Strahlungsverteilersonde 4 auf dem Aussenprofil 10 des ersten Abschnitts 9 der Innenhülse 3 sitzt. Ist das Aussenprofil 10 der Innenhülse 3 wie dargestellt als Aussensechskant ausgeführt, so ist das Innenprofil 20 (vergleiche Figuren 6c und 9b) der Strahlungsverteilersonde 4 als zu diesem Aussensechskant passenden Innensechskant ausgeführt.

Das Vorsehen eines Aussenprofils 10 an einem ersten Abschnitt 9 der Innenhülse 3 und eines dazu passenden Innenprofils 20 an der Strahlungsverteilersonde 4 stellt eine Verdrehsicherung dar. Solange die Innenhülse 3 über ihr Aussenprofil 10 mit der Strahlungsverteilersonde 4 bzw. deren Innenprofil 20 verbunden ist, kann die Strahlungsverteilersonde 4 nicht durch Drehen von der Aussenhülse 2 entfernt werden. Vor dem Entfernen der Strahlungsverteilersonde 4 von der Aussenhülse 2 muss somit zuerst die Innenhülse 3 von der Strahlungsverteilersonde 4 gelöst werden, d.h. das Aussenprofil 10 der Innenhülse 3 muss von dem Innenprofil 20 der Strahlungsverteilersonde 4 gelöst werden. Hierzu wird der Verschluss 5, der vorzugsweise über eine Steck- und/oder Schraubverbindung mit der Aussenhülse 2 verbunden ist, von der Aussenhülse 2 gelöst und die Innenhülse 3 aus der Aussenhülse 2 entfernt. Danach kann die Strahlungsverteilersonde 4 von der Aussenhülse 2 gelöst werden, insbesondere durch eine Drehbewegung.

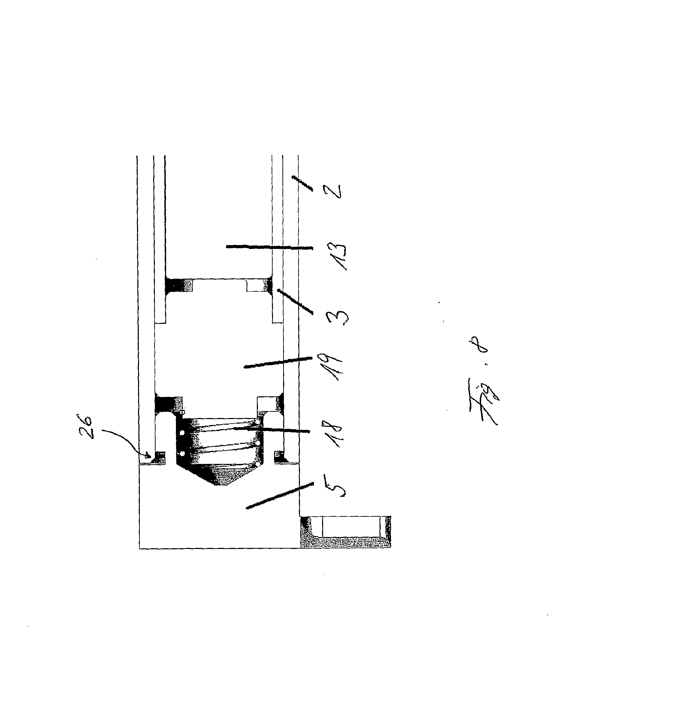

An dem Bereich der Aussenhülse 2, an den die Strahlungsverteilersonde 4 angebracht wird, ist vorzugsweise ein Dichtring 8, der auch als Sicherungsring bezeichnet werden kann, vorgesehen, um die Innenhülse 3 vor Verschmutzung zu schützen. Selbstverständlich kann der Dichtring 8 oder ein zusätzlicher Dichtring bzw. Sicherungsring auch in der Strahlungsverteilersonde 4 selbst angeordnet sein. Zwischen dem Verschluss 5 und der Aussenhülse 2 ist vorzugsweise ein weiterer Dichtring 26 vorgesehen (vgl. Figur 8).

Durch die Verdrehsicherung zwischen der Innenhülse 3 und der Strahlungsverteilersonde 4 wird sichergestellt, dass die Strahlungsverteilersonde 4 nicht vor der Entfernung der Innenhülse 3 aus der Aussenhülse 2 von der Aussenhülse 2 abgenommen wird. Auf diese Weise wird gewährleistet, dass bei einem Reinigen und Sterilisieren der tragbaren Bestrahlungsanordnung 1 die Innenhülse 3, falls sterilisierbar, nicht in Kontakt mit Wasserdampf und/oder anderen Reinigungsmitteln kommt; da die Innenhülse 3 vorzugsweise die Lichtquelle, die Stromversorgung für die Lichtquelle und weitere elektronische Bauelemente zum Ansteuern der Lichtquelle enthält. Durch den Dichtungsring 8 wird die Innenhülse noch weiter gegen eine Verschmutzung geschützt.

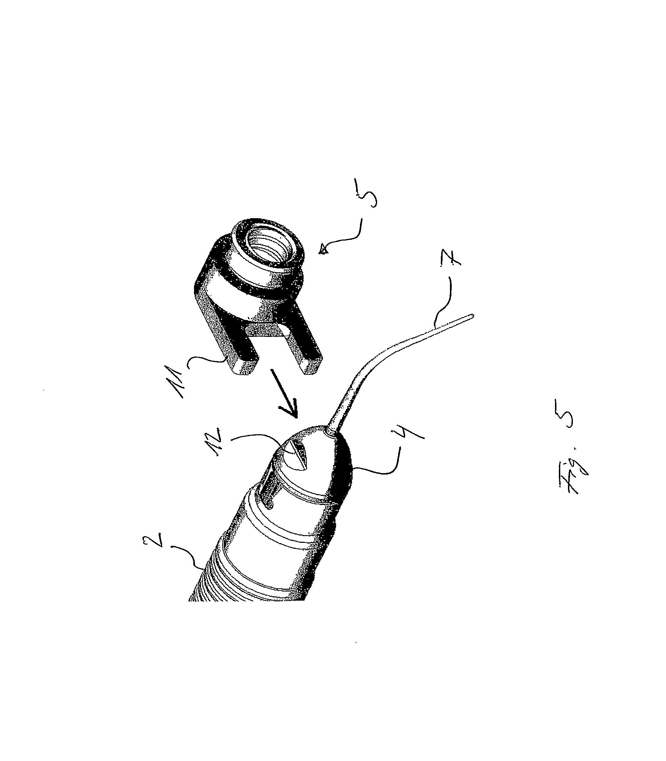

Der Verschluss 5 weist vorzugsweise eine Löseeinrichtung 11 zum Lösen der Strahlungsverteilersonde 4 von der Aussenhülse 2 auf. Wie in Figur 5 dargestellt ist die Löseeinrichtung 11 bevorzugt U-förmig ausgestaltet. Ferner weist die Strahlungsverteilersonde 4 an ihrer Aussenseite bevorzugt eine längliche Ausbuchtung 12 auf, die derart ausgestaltet ist, dass die Löseeinrichtung 11 an sie zum Lösen der Strahlungsverteilervorrichtung 4 angreifen kann. Das heisst, die U-förmige Löseeinrichtung 11 wird in bzw. an der länglichen Ausbuchtung 12 platziert. Durch Drehen des Verschlusses 5 kann dann die Strahlungsverteilersonde 4 von der Aussenhülse 2 gelöst bzw. "abgedreht" werden. Zusätzlich kann durch diese Ausgestaltung der Löseeinrichtung 11 ein unbeabsichtigtes Rollen der erfindungsgemässen Bestrahlungsanordnung 1 auf bzw. von einer Oberfläche - beispielsweise auf bzw. von einem Tisch - verhindert und die Bestrahlungsanordnung 1 kann so vor Beschädigungen geschützt werden.

Die Figuren 6 bis 9 zeigen Querschnittsdarstellungen und eine Rückansicht der erfindungsgemässen, tragbaren Bestrahlungsanordnung 1, wobei in Figur 6a die Rückansicht den Verschluss 5 mit der U-förmigen Löseeinrichtung 11 zeigt.

In der Innenhülse 3 ist vorzugsweise sondenseitig die Lichtquelle 14 angeordnet, bei der es sich vorzugsweise um eine Laserdiode handelt, der bevorzugt eine Linse zugeordnet ist. Zur Stromversorgung der Lichtquelle 14 ist in der Innenhülse 3 eine Stromquelle 13, bei der es sich vorzugsweise um eine Batterie oder einen Akkumulator, bevorzugt um zwei Batterien bzw. Akkumulatoren, handelt, angeordnet. Ferner sind elektronische Bauelemente 15 zur Steuerung bzw. Ansteuerung der Lichtquelle 14 vorgesehen, die vorzugsweise in axialer Richtung zwischen der Stromquelle 13 und der Lichtquelle 14 angeordnet sind. Die elektrischen Bauelemente 15 weisen einen Kontakt 16 für das Bedienelement 6 auf.

In der Innenhülse 3 ist eine Öffnung 17 (vergleiche Figur 2) vorgesehen, über die das Bedienelement mit dem Kontakt 16 und auf diese Weise mit den elektronischen Bauelementen 15 in Kontakt bzw. Wirkverbindung treten kann, wenn die Strahlungsverteilersonde 4 an die Aussenhülse 2 angebracht ist. Der Kontakt 16 ist vorzugsweise als Taster und das Bedienelement 6 ist vorzugsweise als Druckknopf ausgeführt, sodass bei Drücken des Bedienelements 6 der Taster 16 gedrückt und auf diese Weise ein Stromkreis für die Stromversorgung der Lichtquelle 14 geschlossen und die Lichtquelle 14 mit Strom versorgt wird.

Es ist eine Federung 18 zur federnden Lagerung der Innenhülse 3 vorgesehen. Die Federung 18 ist bevorzugt zwischen dem Verschluss 5 und der Innenhülse 3 in axialer Richtung vorgesehen und als Feder ausgeführt, wobei zwischen der Federung 18 und der Innenhülse 3 zusätzlich ein Verschlusspfropfen 19 zum eigenständigen Verschliessen des der Strahlungsverteilersonde 4 gegenüber liegenden Endes der Innenhülse 3 (vergleiche Figur 8) lösbar angeordnet ist. Selbstverständlich kann die Innenhülse 3 auch auf andere Weise lösbar verschlossen sein, sodass die Stromquelle 13 gegebenenfalls ausgetauscht werden kann. Zwischen der Stromquelle 13 und den elektronischen Bauelementen 15 ist vorzugsweise ebenfalls eine Federung 21, insbesondere eine Feder, vorgesehen, um den elektrischen Kontakt zwischen der Stromquelle 13 und den elektronischen Bauelementen 15 zu verbessern.

Als Lichtquelle 14 kann auch eine Lichtwellenleiter-Faser eingesetzt werden (nicht dargestellt), wobei in diesem Falle auf die Stromquelle 13 und die elektronischen Bauelemente 15 verzichtet werden kann. Die Bestrahlungsanordnung 1 ist dann als faseroptisches Handstück ausgebildet, bei dem nur die Aussenhülse 2 sterilisiert werden muss und nicht ihr Innenleben.

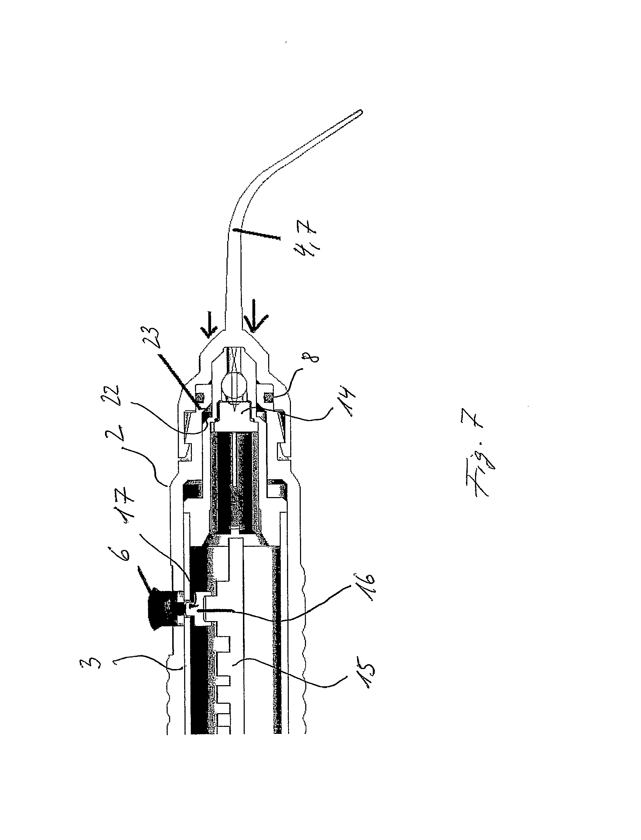

Figur 6c zeigt den in Figur 6b mit "C" bezeichneten Ausschnitt in vergrösserter Darstellung. Die Strahlungsverteilersonde 4 ist separat, d.h. nicht an die Aussenhülse 2 angebracht, dargestellt. Durch die Federung 18 ist die Innenhülse 3 derart in der Aussenhülse 2 positioniert, dass der erste, sondenseitige Abschnitt 9 zumindest teilweise aus dem sondenseitigen Ende der Aussenhülse herausragt. An den ersten Abschnitt 9 der Innenhülse 3 schliesst sich vorzugsweise ein zweiter Abschnitt 22 mit einem grösseren Aussenradius, der sich insbesondere konisch ausweitet, an. Die Aussenhülse 2 weist vorzugsweise an ihrer Innenseite einen Anschlag 23 für den zweiten Abschnitt 22 der Innenhülse 4 auf. Durch die Federung 18 wird der zweite Abschnitt 22 der Innenhülse 3 gegen den Anschlag 23 der Aussenhülse 2 gedrückt, wenn die Strahlungsverteilersonde 4 nicht auf die Aussenhülse 2 aufgebracht ist. Bei dieser Stellung der Innenhülse 3 im Verhältnis zur Aussenhülse 2 ist das Bedienelement 6 in axialer Richtung so weit von der in der Innenhülse 3 vorhandenen Öffnung 17 (vergleiche Figuren 2 und 7) beabstandet bzw. entfernt, dass es durch die Öffnung 17 nicht in Wirkverbindung mit den elektronischen Bauelementen 15 bzw. deren Kontakt 16 treten kann.

Wird die Strahlungsverteilersonde 4 nun auf die Aussenhülse 2 aufgebracht, so schiebt sie den ersten Abschnitt 9 der Innenhülse 3 zumindest teilweise gegen die Federkraft der Federung 18 in die Aussenhülse 2 zurück, sodass der zweite Abschnitt 22 der Innenhülse 3 nicht mehr an dem Anschlag 23 der Aussenhülse 2 anliegt und sich das Bedienelement 6 nun über der Öffnung 17 der Innenhülse 3 befindet und somit durch seine Betätigung in Wirkverbindung mit den elektronischen Bauelementen 15 bzw. deren Kontakt 16 treten kann (vergleiche Figur 7). Die Pfeile in der Figur 7 markieren die Bewegungsrichtung der Strahlungsverteilersonde 4 beim Anbringen an die Aussenhülse 2 der tragbaren Bestrahlungsanordnung 1.

Ist die Strahlungsverteilersonde 4 nicht an die Aussenhülse 2 angebracht, so sperrt die Innenhülse 3 das Bedienelement 6, sodass es sich nicht mehr betätigen lässt, da sich die Öffnung 17 der Innenhülse 3 nicht mehr unter dem Bedienelement 6 befindet. Die tragbare Bestrahlungsanordnung 1 kann also nicht ohne angebrachte Strahlungsverteilersonde 4 in Betrieb genommen werden und es kann in dem Fall, in dem die Strahlungsverteilersonde 4 nicht angebracht ist, keine Bestrahlung austreten, sodass eine sichere Benutzung der tragbaren Bestrahlungsanordnung 1 gewährleistet ist.

Die Figuren 9a und 9b zeigen die in den Figuren 6b und 6c dargestellte Bestrahlungsanordnung, wobei zusätzlich ein Sensor 24 vorgesehen ist, der insbesondere in bzw. an der Aussenhülse 2 angeordnet ist. Der Sensor 24 ist derart ausgebildet, dass er erkennt, ob eine Strahlungsverteilersonde 4 an der Aussenhülse 2 angebracht ist. Er ist über nicht näher bezeichnete elektrische Leitungen mit den elektrischen Bauelementen 15 verbunden. Der Sensor 24 ist vorzugsweise als Drucksensor, insbesondere als Taster, ausgebildet. Erkennt der Sensor 24 das Angebrachtsein einer Strahlungsverteilersonde 4, so gibt er ein entsprechendes Signal ab bzw. verändert seine Stellung derart - falls er als Schalter bzw. Taster ausgebildet ist -, dass als Folge hiervon der Stromkreis zwischen der Stromquelle 13 und der Lichtquelle 14 bei Betätigung des Bedienelements 6 geschlossen wird und somit die tragbare Bestrahlungsanordnung 1 durch Betätigung des Bedienelements 6 eingeschaltet werden kann.

Ist der Sensor 24 als Drucksensor ausgeführt, so weist die Strahlungsverteilersonde 4 vorzugsweise eine Ausbuchtung 25 auf, die auf den Sensor 24 drückt, wenn die Strahlungsverteilersonde 4 auf die Aussenhülse 2 aufgebracht ist. Dies führt beim Sensor 24 zu einem Erkennen der aufgebrachten Strahlungsverteilersonde 4. Der Sensor 24 ist vorzugsweise am Umfang der Aussenhülse 2 angeordnet. Alternativ oder zusätzlich kann der bzw. ein Sensor 24 auch an der Stirnseite der Aussenhülse 2 angeordnet sein. Insbesondere im letzteren Fall ist eine Ausbuchtung 25 an der Strahlungsverteilersonde 4 nicht unbedingt notwendig.

Selbstverständlich kann zum sicheren Betreiben der erfindungsgemässen tragbaren Bestrahlungsanordnung 1 auch nur ein Sensor 24 vorgesehen sein, der wie oben beschrieben ausgebildet ist. Das heisst, es kann grundsätzlich davon abgesehen werden, dass die Innenhülse 3 bei nicht angebrachter Strahlungsverteilersonde 4 weiter als bei angebrachter Strahlungsverteilersonde 4 aus der Aussenhülse 2 herausragt, sodass das Bedienelement 6 bei nicht angebrachter Strahlungsverteilersonde 4 nicht über die Öffnung 17 mit den elektrischen Bauelementen 15 in Wirkverbindung treten kann, da bereits über den Sensor 24 sichergestellt wird, dass nur bei angebrachter Strahlungsverteilersonde 4 ein Stromfluss von der Stromquelle 13 zur Lichtquelle 14 möglich ist.

Die Ausgestaltung gemäss Figur 9 gewährleistet jedoch eine höhere Sicherheit für den Benutzer, da erst durch Anbringen der Strahlungsverteilersonde 4 die Innenhülse 3 so weit in die Aussenhülse 2 zurück geschoben wird, dass das Bedienelement 6 bei Betätigung über die Öffnung 17 mit der Lichtquelle 14 in Wirkverbindung treten kann und noch zusätzlich das Angebrachtsein der Strahlungsverteilersonde 4 über den Sensor 24 detektiert wird.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Tragbare Bestrahlungsanordnung mit einer Lichtquelle (14), umfassend eine Aussenhülse (2), eine Innenhülse (3) und eine Strahlungsverteilersonde (4), wobei die Innenhülse (3) in der Aussenhülse (2) gelagert ist und die Strahlungsverteilersonde (4) an einem sondenseitigen Ende der Aussenhülse (2) anbringbar ist, wobei die Lichtquelle (14) nur dann einschaltbar ist, wenn die Strahlungsverteilersonde (4) an der Aussenhülse (2) angebracht ist,
**dadurch gekennzeichnet,**
**dass** die Lichtquelle ein Laser bzw. eine Laserdiode ist, oder als Lichtwellenleiter ausgeführt ist.

2. Tragbare Bestrahlungsanordnung nach Anspruch 1, **dadurch gekennzeichnet, dass** eine Federung (18) vorgesehen ist, die in axialer Richtung auf die Innenhülse (3) wirkt, sodass ein erster Abschnitt (9) der Innenhülse (3) zumindest teilweise aus dem sondenseitigen Ende der Aussenhülse (2) herausragt, und dass die Strahlungsverteilersonde (4) derart ausgebildet ist, dass sie im angebrachten Zustand einen aus der Aussenhülse (2) herausragenden Teil des ersten Abschnitts (9) der Innenhülse (3) gegen die Federkraft der Federung (18) zumindest teilweise in die Aussenhülse (2) drückt.

3. Tragbare Bestrahlungsanordnung nach Anspruch 2, **dadurch gekennzeichnet, dass** sich an den ersten Abschnitt (9) der Innenhülse (3) ein zweiter Abschnitt (22) mit einem grösseren Aussenradius, insbesondere ein sich konisch aufweitender Abschnitt, anschliesst, dass die Aussenhülse (2) einen Anschlag (23) für den zweiten Abschnitt (22) der Innenhülse (3) aufweist und dass bei entfernter Strahlungsverteilersonde (4) die Federung (18) den zweiten Abschnitt (22) der Innenhülse (3) gegen den Anschlag (23) der Aussenhülse (2) drückt.

4. Tragbare Bestrahlungsanordnung nach Anspruch 3, **dadurch gekennzeichnet, dass** ein Bedienelement (6) zum Einschalten der Lichtquelle (14) vorgesehen ist.

5. Tragbare Bestrahlungsanordnung nach Anspruch 4, **dadurch gekennzeichnet, dass** das Bedienelement (6) an der Aussenhülse (2) vorgesehen ist und dass die Innenhülse (3) eine Öffnung (17) aufweist, wobei der axiale Abstand zwischen dem Bedienelement (6) und der Öffnung (17) derart gewählt ist, dass nur bei angebrachter Strahlungsverteilersonde (4) das Bedienelement (6) durch die Öffnung (17) in Wirkverbindung mit der Lichtquelle (14) treten kann.

6. Tragbare Bestrahlungsanordnung nach Anspruch 4, **dadurch gekennzeichnet, dass** das Bedienelement (6) als Fernbedienung, insbesondere als Fussschalter, ausgebildet ist.

7. Tragbare Bestrahlungsanordnung nach Anspruch 5 oder 6, **dadurch gekennzeichnet, dass** das Bedienelement (6) als Druckknopf, als Berührfeld, als Schalter, als Taster, kapazitiv, induktiv und/oder optisch ausgebildet ist.

8. Tragbare Bestrahlungsanordnung nach einem der vorhergehenden Ansprüche, **dadurch gekennzeichnet, dass** in bzw. an der Aussenhülse (2) ein Sensor (24) vorgesehen ist, der derart ausgebildet ist, dass er ein Angebrachtsein der Strahlungsverteilersonde (4) erkennt.

9. Tragbare Bestrahlungsanordnung nach einem der vorhergehenden Ansprüche, **dadurch gekennzeichnet, dass** die Innenhülse (3) derart mit der Strahlungsverteilersonde (4) in Kontakt steht, dass vor einem Entfernen der Strahlungsverteilersonde (4) von der Aussenhülse (2) die Innenhülse (3) von der Strahlungsverteilersonde (4) gelöst werden muss.

10. Tragbare Bestrahlungsanordnung nach Anspruch 9, **dadurch gekennzeichnet, dass** die Innenhülse (3) drehfest in der Aussenhülse (2) gelagert ist.

11. Tragbare Bestrahlungsanordnung nach Anspruch 9 oder 10, **dadurch gekennzeichnet, dass** die Innenhülse (3) an einem ersten, sondenseitigen Abschnitt (9) ein Aussenprofil (10) und die Strahlungsverteilersonde (4) ein dazu passendes Innenprofil (20) ausweist, wobei das Innenprofil (20) bei angebrachter Strahlungsverteilersonde (4) auf dem Aussenprofil (10) sitzt.

12. Tragbare Bestrahlungsanordnung nach Anspruch 11, **dadurch gekennzeichnet, dass** das Aussenprofil (10) der Innenhülse (3) als Aussensechskant und das Innenprofil (20) der Strahlungsverteilersonde (4) als dazu passender Innensechskant ausgeführt sind.

13. Tragbare Bestrahlungsanordnung nach einem der vorhergehenden Ansprüche, **dadurch gekennzeichnet, dass** an dem dem sondenseitigen Ende der Aussenhülse (2) gegenüberliegenden Ende ein Verschluss (5) vorgesehen ist, der eine Löseeinrichtung (11) zum Lösen der Strahlungsverteilersonde (4) von der Aussenhülse (2) aufweist.

14. Tragbare Bestrahlungsanordnung nach Anspruch 13, **dadurch gekennzeichnet, dass** die Löseeinrichtung (11) U-förmig ausgestaltet ist und die Strahlungsverteilersonde (4) an ihrer Aussenseite eine längliche Ausbuchtung (12) aufweist, die derart ausgestaltet ist, dass die Löseeinrichtung (11) an sie zum Lösen der Strahlungsverteilersonde (4) angreifen kann.

15. Tragbare Bestrahlungsanordnung nach Anspruch 13 oder 14, **dadurch gekennzeichnet, dass** die Löseeinrichtung (11) derart ausgestaltet ist, dass sie ein Rollen der Bestrahlungsanordnung (1) auf bzw. von einer Oberfläche verhindert.

## Claims

1. Portable irradiation arrangement with a light source (14), comprising an outer shell (2), an inner shell (3) and an irradiation distribution probe (4), wherein the inner shell (3) is supported inside the outer shell (2) and the irradiation distribution probe (4) is attachable to a probe-sided end of the outer shell (2), wherein the light source (14) can only be switched on if the irradiation distribution probe (4) is attached to the outer shell (2),
**characterized in that** the light source is a laser or a laser diode, respectively, or **in that** it is formed as optical waveguide.

2. Portable irradiation arrangement according to claim 1, **characterized in that** a spring (18) is provided, acting upon the inner shell (3) in axial direction such that a first section (9) of the inner shell (3) protrudes at least partially out of probe-sided end of the outer shell (2), and **in that** the irradiation distribution probe (4) is formed in such a way that it presses a part of the first section (9) of the inner shell (3), which protrudes out of the outer shell (2), at least partially into the outer shell (2) against the spring force of the spring (18).

3. Portable irradiation arrangement according to claim 2, **characterized in that** a second section (22) with a larger outer radius, particularly a section expanding conically, follows the first section (9) of the inner shell (3), **in that** the outer shell (2) has an abutment (23) for the second section (22) of the inner shell (3) and **in that** the spring (18) presses the second section (22) of the inner shell (3) against the abutment (23) of the outer shell (2) in case of removed irradiation distribution probe (4).

4. Portable irradiation arrangement according to claim 3, **characterized in that** an operating element (6) is provided for switching on the light source (14).

5. Portable irradiation arrangement according to claim 4, **characterized in that** the operating element (6) is provided at the outer shell (2) and **in that** the inner shell (3) has an opening (17), wherein the axial distance between the operating element (6) and the opening (17) is chosen in such a way that the operating element (6) can have operative connection with the light source (14) through the opening (17) only in case of attached irradiation distribution probe (4).

6. Portable irradiation arrangement according to claim 4, **characterized in that** the operating element (6) is formed as remote control, particularly as foot switch.

7. Portable irradiation arrangement according to claim 5 or 6, **characterized in that** the operating element (6) is formed as push knob, as touch field, as switch, as button, capacitive, inductive and/or optical.

8. Portable irradiation arrangement according to one of the preceding claims, **characterized in that** a sensor (24) is provided in the outer shell (2) or at the outer shell (2), respectively, being formed in such a way that it detects an attached-state of the irradiation distribution probe (4).

9. Portable irradiation arrangement according to one of the preceding claims, **characterized in that** the inner shell (3) is in contact with the irradiation distribution probe (4) in such a way that the inner shell (3) has to be detached from the irradiation distribution probe (4) before removing the irradiation distribution probe (4) from the outer shell (2).

10. Portable irradiation arrangement according to claim 9, **characterized in that** the inner shell (3) is supported in a rotation-proof way inside the outer shell (2).

11. Portable irradiation arrangement according to claim 9 or 10, **characterized in that** the inner shell (3) has an outer profile (10) at a first probe-sided section (9) and the irradiation distribution probe (4) has an inner profile (20) fitting to the outer profile, wherein the inner profile (20) sits on the outer profile (10) when the irradiation distribution probe (4) is attached.

12. Portable irradiation arrangement according to claim 11, **characterized in that** the outer profile (10) of the inner shell (3) is formed as external hexagon and the inner profile (20) of the irradiation distribution probe (4) is formed as fitting internal hexagon.

13. Portable irradiation arrangement according to one of the preceding claims, **characterized in that** a closure (5) is provided at the opposite end of the outer shell (2) with respect to the probe-sided end, having a detachment device (11) for detaching the irradiation distribution probe (4) from the outer shell (2).

14. Portable irradiation arrangement according to claim 13, **characterized in that** the detachment device (11) is U-shaped and the irradiation distribution probe (4) has an elongated protrusion (12) on its outer face, which is formed in such a way that the detachment device (11) can engage it for detaching the irradiation distribution probe (4).

15. Portable irradiation arrangement according to claim 13 or 14, **characterized in that** the detachment device (11) is formed in such a way that it avoids a rolling of the irradiation arrangement (1) on a surface or from a surface, respectively.

## Revendications

1. Arrangement d'irradiation portable avec une source de lumière (14), comprenant une coque extérieure (2), une coque intérieure (3) et une sonde de distribution d'irradiation (4), la coque intérieure (3) étant supportée à l'intérieur de la coque extérieure (2) et la sonde de distribution d'irradiation (4) étant rattachable à une extrémité côté sonde de la coque extérieure (2), la source de lumière (14) étant permise d'être allumée uniquement si la sonde de distribution d'irradiation (4) est attachée à la coque extérieure (2),
**caractérisé en ce que** la source de lumière est un laser ou bien une diode laser ou un guide d'ondes optiques.

2. Arrangement d'irradiation portable selon la revendication 1, **caractérisé en ce qu'**un ressort (18) est prévu, actionnant sur la coque intérieure (3) en direction axiale, de sorte qu'une première section (9) de la coque intérieure (3) fait saille au moins partiellement de l'extrémité côté sonde de la coque extérieure (2), et **en ce que** la sonde de distribution d'irradiation (4) est formée de sorte qu'elle presse une partie de la première section (9) de la coque intérieure (3), qui fait saille de la coque extérieure (2), au moins partiellement dans la coque extérieure (2) contre la force de ressort du ressort (18).

3. Arrangement d'irradiation portable selon la revendication 2, **caractérisé en ce qu'**une deuxième section (22) avec un radius externe supérieur, particulièrement une section s'étendant de manière conique, suit la première section (9) de la coque intérieure (3), **en ce que** la coque extérieure (2) a une butée (23) pour la deuxième section (22) de la coque intérieure (3) et **en ce que** le ressort (18) presse la deuxième section (22) de la coque intérieure (3) contre la butée (23) de la coque extérieure (2) en cas d'une sonde de distribution d'irradiation (4) enlevée.

4. Arrangement d'irradiation portable selon la revendication 3, **caractérisé en ce qu'**un élément d'opération (6) est prévu pour allumer la source de lumière (14).

5. Arrangement d'irradiation portable selon la revendication 4, **caractérisé en ce que** l'élément d'opération (6) est prévu sur la coque extérieure (2) et **en ce que** la coque intérieure (3) a une ouverture (17), la distance axiale entre l'élément d'opération (6) et l'ouverture (17) étant choisie de sorte que l'élément d'opération (6) puisse avoir une connexion d'opération avec la source de lumière (14) à travers l'ouverture (17) uniquement quand la sonde de distribution d'irradiation (4) est attachée.

6. Arrangement d'irradiation portable selon la revendication 4, caractérisé en ce l'élément d'opération (6) est une télécommande, particulièrement un interrupteur à pied.

7. Arrangement d'irradiation portable selon la revendication 5 ou 6, **caractérisé en ce que** l'élément d'opération (6) est un bouton-poussoir, un pavé tactile, un interrupteur, un bouton, capacitif, inductif et/ou optique.

8. Arrangement d'irradiation portable selon l'une des revendications précédentes, **caractérisé en ce qu'**un capteur (24) est prévu dans ou bien à la coque extérieure (2), étant formé de sorte qu'il détecte un état attaché de la sonde de distribution d'irradiation (4).

9. Arrangement d'irradiation portable selon l'une des revendications précédentes, **caractérisé en ce que** la coque intérieure (3) est en contact avec la sonde de distribution d'irradiation (4) de sorte que la coque intérieure (3) doit être détachée de la sonde de distribution d'irradiation (4) avant enlever la sonde de distribution d'irradiation (4) de la coque extérieure (2).

10. Arrangement d'irradiation portable selon la revendication 9, **caractérisé en ce que** la coque intérieure (3) est supportée de manière antirotation dans la coque extérieure (2).

11. Arrangement d'irradiation portable selon la revendication 9 ou 10, **caractérisé en ce que** la coque intérieure (3) a un profile extérieur (10) à la section côté sonde (9) et la sonde de distribution d'irradiation (4) a un profile intérieur (20) adapté au profile extérieur, le profile intérieur (20) étant posé sur le profile extérieur (10) quand la sonde de distribution d'irradiation (4) est attachée.

12. Arrangement d'irradiation portable selon la revendication 11, **caractérisé en ce que** le profile extérieur (10) de la coque intérieure (3) est formé comme six-pans extérieurs et le profile intérieur (20) de la sonde de distribution d'irradiation (4) est formé comme un six-pans intérieurs adapté au six-pans extérieur.

13. Arrangement d'irradiation portable selon l'une des revendications précédentes, **caractérisé en ce qu'**un obturateur (5) est prévu de l'extrémité du côté opposé de la coque extérieure (2) par rapport à l'extrémité côté sonde, ayant un dispositif de détachement (11) pour détacher la sonde de distribution d'irradiation (4) de la coque extérieure (2).

14. Arrangement d'irradiation portable selon la revendication 13, **caractérisé en ce que** le dispositif de détachement (11) est en forme de U et la sonde de distribution d'irradiation (4) a une protubérance allongée (12) de son côté extérieur, formée de sorte que le dispositif de détachement (11) puisse l'engager afin de détacher la sonde de distribution d'irradiation (4).

15. Arrangement d'irradiation portable selon la revendication 13 ou 14, **caractérisé en ce que** le dispositif de détachement (11) est formé de sorte qu'il évite un roulement de l'arrangement d'irradiation (1) sur une ou bien d'une surface.
